# EUROPÄISCHE PATENTSCHRIFT

(11) **EP 4 028 086 B1**
(45) Veröffentlichungstag und Bekanntmachung des Hinweises auf die Patenterteilung: **05.11.2025**
(21) Anmeldenummer: 20768025.7
(22) Anmeldetag: 07.09.2020
(51) Int. Cl.: A61M 15/06

(54) **VERDAMPFERKARTUSCHE SOWIE INHALATOR MIT EINER SOLCHEN VERDAMPFERKARTUSCHE**
VAPORIZER CARTRIDGE AND INHALER COMPRISING SUCH A VAPORIZER CARTRIDGE
CARTOUCHE D'ÉVAPORATEUR ET INHALATEUR COMPRENANT UNE TELLE CARTOUCHE D'ÉVAPORATEUR

(30) Priorität: 11.09.2019 DE 102019124411
(43) Veröffentlichungstag der Anmeldung: 20.07.2022
(73) Patentinhaber: Körber Technologies GmbH, 21033 Hamburg (DE)
(72) Erfinder: KLEINE-WÄCHTER, Michael, 23881 Lankau (DE); KOCK, Lennart, 22301 Hamburg (DE)
(74) Vertreter: Stork Bamberger Patentanwälte PartmbB
(86) Internationale Anmeldenummer: PCT/EP2020/074906
(87) Internationale Veröffentlichungsnummer: WO 2021/048039

(56) Entgegenhaltungen:
- EP-A1- 2 599 514
- WO-A1-2018/083007
- DE-A1- 102017 123 868

## Beschreibung

Die Erfindung betrifft eine Verdampferkartusche geeignet für ein Inhalator, umfassend mindestens einen Vorratstank zum Aufnehmen und Bevorraten einer Flüssigkeit und mindestens einen Hohlkörper mit einem durchgängigen Luftströmungskanal, wobei der Vorratstank mindestens eine Zugangsöffnung zum Luftströmungskanal aufweist und im Bereich jeder Zugangsöffnung eine sich über die gesamte Zugangsöffnung erstreckende Verdampfereinheit angeordnet ist, die ein dem Vorratstank zugewandtes Dochtorgan und ein dem Luftströmungskanal zugewandtes Heizorgan aufweist, wobei die Verdampfereinheit flüssigkeitspermeabel ausgebildet ist, derart, dass Flüssigkeit mindestens initial kapillar aus dem Vorratstank durch die Verdampfereinheit in Richtung des Luftströmungskanals förderbar ist.

Des Weiteren betrifft die Erfindung einen Inhalator laut Anspruch 17, ausgebildet und eingerichtet zum Inhalieren von mit Wirkstoffen angereichertem Dampf, umfassend einen mindestens eine elektronische Steuereinheit und eine Energiequelle umfassenden Kartuschenträger sowie eine Verdampferkartusche.

Solche Verdampferkartuschen und Inhalatoren kommen in der Genussmittelindustrie, hier insbesondere im Zusammenhang mit einer elektronischen Zigarette, der so genannten E-Zigarette, sowie im medizinischen Bereich zum Einsatz, um fluide Genussmittel und/oder fluide medizinische Produkte in Dampfform und/oder als Aerosole inhalieren zu können, siehe DE102017123868.

Beim Konsumieren saugt üblicherweise eine Person an einem Mundstück des Inhalators, wodurch in dem Luftströmungskanal ein Saugdruck entsteht, der einen Luftstrom durch den Luftströmungskanal erzeugt. Der Luftstrom kann aber auch maschinell z.B. durch eine Pumpe erzeugt werden. In dem Luftströmungskanal wird dem Luftstrom eine von der Verdampfereinheit bereitgestellte verdampfte Flüssigkeit zugegeben, um der konsumierenden Person ein Aerosol oder ein Aerosol-Dampf-Gemisch zu verabreichen. Die Flüssigkeit ist an der oder in der Verdampferkartusche bevorratet. Als Flüssigkeit kommen unterschiedliche Mischungen mit verschiedenen Bestandteilen gleicher oder unterschiedlicher Dampfdichten zum Einsatz. Eine typische Mischung für den Einsatz in einer E-Zigarette weist z.B. Bestandteile von Glycerin und Propylenglycol auf, ggf. angereichert um Nikotin und/oder nahezu beliebige Geschmacksstoffe. Für den Einsatz im medizinischen oder therapeutischen Bereich, z.B. zur Inhalation von Asthma-Präparaten, kann die Mischung entsprechend medizinische Bestandteile und Wirkstoffe aufweisen.

Die einzelnen Bestandteile der Verdampferkartusche, nämlich der Vorratstank, der Hohlkörper und die Verdampfereinheit, können in einem gemeinsamen Bauteil zusammengefasst sein, wobei dieses Bauteil dann ein Einwegartikel ist, der für eine endliche Anzahl von Inhalationszügen durch eine konsumierende Person ausgelegt ist und zusammen mit einem Kartuschenträger als wiederverwendbarem Mehrwegartikel, der mindestens eine elektronische Steuereinheit und eine Energiequelle umfasst, einen Inhalator bildet. Die Verdampferkartusche kann jedoch auch erst durch das Zusammenfügen mehrerer Bauteile gebildet sein, wobei einzelne Bauteile, nämlich insbesondere der Hohlkörper und die Verdampfereinheit, in dem Kartuschenträger als Mehrwegartikel angeordnet sind, und der Vorratstank als separates Bauteil den Einwegartikel bildet. Letztlich lässt sich der Inhalator durch Austausch des Einwegartikels, der üblicherweise die Flüssigkeit beinhaltet, variabel einsetzen.

Entsprechend sind der Einwegartikel und der Mehrwegartikel lösbar miteinander verbunden. Der Kartuschenträger als Mehrwegartikel umfasst üblicherweise mindestens eine elektronische Steuereinheit und eine Energiequelle. Die Energiequelle kann z.B. eine elektrochemische Einwegbatterie oder ein wiederaufladbarer elektrochemischer Akku, z.B. ein Li-lonen-Akku sein, mittels dem das Heizorgan über elektrische Kontakte der Verdampfereinheit mit Energie versorgt wird. Die elektronische und/oder elektrische Steuereinheit dient zum Steuern der Verdampfereinheit innerhalb der Verdampferkartusche. Der Kartuschenträger kann aber auch Bestandteile der Verdampferkartusche umfassen. Der Einwegartikel kann als Ansteckteil an den Mehrwegartikel ansteckbar oder als Einsetzteil in den Mehrwegartikel einsetzbar ausgebildet sein. Anstelle einer Steckverbindung sind auch Schraubverbindungen, Schnappverbindungen oder andere Schnellverbindungen einsetzbar. Mit der Verbindung von Einwegartikel und Mehrwegartikel wird eine mechanische und elektrische Kopplung zur Bildung eines funktionsbereiten Inhalators hergestellt.

Die zentrale und letztlich die Nutzung (z.B. als E-Zigarette oder als medizinischer Inhalator) bestimmende Komponente ist der Vorratstank als Bestandteil der Verdampferkartusche. Diese beinhaltet in der Regel den Vorratstank mit einer von der Person gewählten, gewünschten und/oder benötigten Flüssigkeit bzw. ein Flüssigkeitsgemisch (im Folgenden auch allgemein als Fluid bezeichnet) sowie den den Strömungskanal bildenden Hohlkörper und die Verdampfereinheit. Das Fluid ist in dem Vorratstank der Verdampferkartusche bevorratet. Mittels der flüssigkeitspermeablen Verdampfereinheit wird das Fluid aus dem Vorratstank aufgrund zumindest initial kapillarer Förderung durch das Dochtorgan und das Heizorgan geleitet. Die von einer Energiequelle erzeugte Spannung, die an dem Heizorgan angelegt wird, führt zu einem Stromfluss im Heizorgan, das vorzugsweise ein flächiges und flaches, z.B. im Wesentlichen aus Silizium bestehendes oder Silizium oder p- oder n-dotiertes Silizium aufweisendes MEMS-Bauteil (Micro-Electro-Mechanical-System-Bauteil) ist. Aufgrund des Heizwiderstandes, vorzugsweise des Ohm'schen Widerstands des Heizorgans, führt der Stromfluss zu einer Erhitzung des Heizorgans und letztlich zu einer Verdampfung des in der Verdampfereinheit befindlichen Fluids. Der auf diese Weise erzeugte Dampf und/oder Aerosol entweichen aus der Verdampfereinheit in Richtung des Luftströmungskanals und werden als Dampfzugabe der Luftströmung beigemischt. Das Fluid hat damit einen vorgegebenen Weg mit einer vorgegebenen Strömungsrichtung, nämlich als Fluid durch das Dochtorgan an das und durch das Heizorgan und dampfförmig aus dem Heizorgan in den Luftströmungskanal. In dem Luftströmungskanal wird das verdampfte Fluid durch den Luftstrom mitgerissen, wobei sich Dampf/Nebel und/oder Aerosol bildet, wenn der Luftströmungskanal mit einem Druck/Unterdruck beaufschlagt wird, indem z.B. eine konsumierende Person an dem Luftströmungskanal saugt oder eine Pumpe einen Luftstrom durch den Luftströmungskanal fördert.

Damit das Fluid aus dem Vorratstank nicht direkt in den Luftströmungskanal fließt, deckt die Verdampfereinheit den Zugang vom Vorratstank zum Luftströmungskanal vollständig ab. Vollständig abgedeckt bedeutet in diesem Zusammenhang, dass die Flüssigkeit zwingend durch die Verdampfereinheit geführt ist, so dass das Fluid nicht direkt vom Vorratstank in den Luftströmungskanal gelangen kann, sondern den "Umweg" über das Dochtorgan und das Heizorgan nehmen muss. Das Dochtorgan dient zum einen zum Zwischenspeichern von Fluid, um insbesondere bei nahezu entleertem Vorratstank noch ausreichend Fluid für wenige Züge am Inhalator zur Verfügung zu stellen. Das Dochtorgan dient zum anderen insbesondere zum Transport des Fluids vom Vorratstank in Richtung des Heizorgans und wirkt gleichzeitig als eine Art Rückschlagschutz, um den Rücklauf von Fluid und/oder Gas bzw. Dampf in Richtung des Vorratstanks zu unterbinden sowie eine Anreicherung einzelner Bestandteile des Fluids bei höheren Temperaturen zu verhindern.

Die bekannten Verdampferkartuschen weisen den Nachteil auf, dass das zwischengespeicherte Flüssigkeitsvolumen durch das - sehr begrenzte - Volumen des Dochtorgans beschränkt ist. Das führt dazu, dass nur solange Flüssigkeit über das Heizorgan verdampft werden kann, wie Flüssigkeit in dem Dochtorgan vorhanden ist. Wenn die im Dochtorgan zwischengespeicherte Flüssigkeit vollständig verdampft ist, heizt das am Dochtorgan anliegende Heizorgan, ohne dass es mit Flüssigkeit benetzt ist, was zu einem so genannten "Dry puff" führt. Ein "Dry puff" kann zu erhöhten Schadstoffwerten in der Luftströmung des Luftströmungskanals führen und die Funktionalität sowie die Lebensdauer des Heizorgans signifikant reduzieren. Des Weiteren erzeugt ein "Dry-puff" wegen der "verbrannten" Flüssigkeit auch eine unerwünschte Geschmacksveränderung.

Entsprechend ist eine kontinuierliche Versorgung bzw. eine permanente Benetzung des Dochtorgans mit Flüssigkeit aus dem Vorratstank notwendig. Bei bekannten Verdampferkartuschen ist die Versorgung des Dochtorgans mit Flüssigkeit aus dem Vorratstank jedoch stark abhängig von der räumlichen Orientierung der Verdampferkartusche und insbesondere des Vorratstanks. Das Dochtorgan ist räumlich nämlich derart zum Vorratstank ausgebildet und ausgerichtet, dass das Dochtorgan nur bei vollständig gefülltem Vorratstank in direktem Kontakt zur Flüssigkeit steht. Im Umkehrschluss bedeutet das, dass das Dochtorgan bei mindestens teilweise geleertem Vorratstank nicht in allen räumlichen Orientierungen der Verdampferkartusche und insbesondere des Vorratstanks in Kontakt mit der Flüssigkeit steht, beispielsweise wenn das Dochtorgan oberhalb des Flüssigkeitsspiegels im Vorratstank liegt. Durch diese Konstellation ist eine kontinuierliche Versorgung des Dochtorgans zumindest temporär unterbrochen, zumal das Heizorgan - aufgrund des geringen Zwischenspeichervolumens des Dochtorgans - auch nur sehr begrenzt aus dem Dochtorgan mit Flüssigkeit versorgt werden kann, mit dem nachteiligen Effekt des beschriebenen "Dry puff". Zusammenfassend ist die Versorgung des Dochtorgans mit Flüssigkeit aus dem Vorratstank lageabhängig und damit diskontinuierlich.

Der Erfindung liegt somit die Aufgabe zugrunde, eine einfach und kostengünstig herzustellende Verdampferkartusche vorzuschlagen, die zur Verhinderung so genannter "Dry puff"-Ereignisse eine kontinuierliche und zuverlässige Versorgung des Dochtorgans mit Flüssigkeit sicherstellt, und zwar unabhängig von der räumlichen Orientierung der Verdampferkartusche. Die Aufgabe besteht weiterhin darin, einen entsprechenden Inhalator zu schaffen.

Diese Aufgabe wird durch eine Verdampferkartusche der eingangs genannten Art dadurch gelöst, dass der Vorratstank im Inneren konstruktiv in ein Hauptvolumen und mindestens ein Nebenvolumen unterteilt ist, wobei das Hauptvolumen und das Nebenvolumen miteinander flüssigkeitsgekoppelt sind und lediglich das Nebenvolumen in direktem Kontakt zur Verdampfereinheit steht, derart, dass Flüssigkeit aus dem Hauptvolumen ausschließlich und zwangsläufig über das Nebenvolumen mit dem Dochtorgan in Kontakt bringbar ist. Das Hauptvolumen ist ein räumlich allseitig begrenzter Aufnahmeraum. Das Nebenvolumen ist - mit Ausnahme der Flüssigkeitskopplung - ein räumlich allseitig begrenzter Aufnahmeraum, der vorzugsweise kleiner ist als das Hauptvolumen. Flüssigkeitsgekoppelt im Sinne der Erfindung bedeutet, dass ein Flüssigkeitsaustausch zwischen Hauptvolumen und Nebenvolumen stattfinden kann. Direkter Kontakt im Sinne der Erfindung bedeutet, dass das Dochtorgan als Bestandteil der Verdampfereinheit durch das Nebenvolumen gegenüber dem Hauptvolumen quasi abgeschirmt wird, so dass Flüssigkeit aus dem Hauptvolumen nur über das Nebenvolumen an das Dochtorgan gelangen kann. Korrespondierend dazu steht das Hauptvolumen nur in indirektem Kontakt zum Dochtorgan. Letztlich liegt das Dochtorgan im Nebenvolumen, so dass das Dochtorgan unabhängig von Lage/Position/Ausrichtung (= räumliche Orientierung) des Vorratstanks und insbesondere des Hauptvolumens gleichmäßig und kontinuierlich mit Flüssigkeit versorgt wird. Selbst bei einer räumlichen Orientierung der Verdampferkartusche, bei der das Dochtorgan oberhalb des Flüssigkeitsspiegels des Hauptvolumens liegt, wird das Dochtorgan aus dem Nebenvolumen mit Flüssigkeit versorgt, so dass das Trockenfallen des Heizorgans verhindert wird. Dadurch, dass ausschließlich das Nebenvolumen in direktem Kontakt zum Dochtorgan steht, ist die Versorgung des Dochtorgans unabhängig von der räumlichen Orientierung des Hauptvolumens des Vorratstanks sichergestellt, wodurch "Dry puff"-Ereignisse wirksam verhindert werden. Im Übrigen vergrößert das Nebenvolumen das - nur sehr begrenzte - Speichervolumen des Dochtorgans, so dass selbst bei leerem Hauptvolumen eine verbesserte und längere Versorgung des Dochtorgans mit Flüssigkeit für zusätzliche Inhalationszüge sichergestellt ist.

Vorzugsweise ist der Vorratstank im Inneren durch eine Trennwand oder dergleichen in das Hauptvolumen und mindestens ein Nebenvolumen unterteilt, wobei das Nebenvolumen zur Aufnahme von Flüssigkeit aus dem Hauptvolumen mit diesem in Flüssigkeitsverbindung steht. Trennwand im Sinne der Erfindung ist jede konstruktive Komponente, die geeignet ist, das Innenvolumen des Vorratstanks in zwei separate Kammern zu teilen. Die Kammern sind durch mindestens eine Öffnung miteinander verbunden, so dass Flüssigkeit aus dem Hauptvolumen in das dem Dochtorgan zugewandte Nebenvolumen strömen und im Nebenvolumen zwischengespeichert werden kann, um das Dochtorgan kontinuierlich mit Flüssigkeit zu versorgen. Dadurch ist auch das Heizorgan zuverlässiger und vom Hauptvolumen und dessen räumlicher Orientierung unabhängiger mit Flüssigkeit zu versorgen. Beim Befüllen der Verdampferkartusche sowie bei der üblichen Handhabung der Verdampferkartusche, insbesondere als Bestandteil des Inhalators, z.B. durch Kippen, Schwenken oder Drehen, gelangt Flüssigkeit aus dem Hauptvolumen über die oder jede Öffnung in das Nebenvolumen, aus dem das Dochtorgan versorgt wird.

Eine bevorzugte Weiterbildung der Verdampferkartusche ist dadurch gekennzeichnet, dass das Nebenvolumen einen Zwischenspeicher bildet und eine Flüssigkeitsverbindung zwischen dem Hauptvolumen und dem Dochtorgan der Verdampfereinheit herstellt. Der Zwischenspeicher speichert einen Vorrat von Flüssigkeit in unmittelbarer Nähe zum Dochtorgan, so das dessen Versorgung mit Flüssigkeit bzw. dessen Benetzung unabhängig vom Füllstand des Hauptvolumens gewährleistet ist. Das Nebenvolumen bildet eine Art Durchgangskammer für die Flüssigkeit, so dass das Dochtorgan über das Nebenvolumen mit der Flüssigkeit aus dem Hauptvolumen in Flüssigkeitsverbindung steht.

Zweckmäßigerweise schirmt der Zwischenspeicher das Dochtorgan mit Ausnahme mindestens einer Zugangsöffnung zur Herstellung der Flüssigkeitsverbindung zwischen dem Nebenvolumen und dem Hauptvolumen gegenüber dem Hauptvolumen ab. Abschirmen im Sinne der Erfindung bedeutet, dass die Flüssigkeit aus dem Hauptvolumen keinen direkten Zugang zum Dochtorgan hat, sondern zwangsläufig den Weg über das Nebenvolumen nehmen muss.

Eine bevorzugte Ausführungsform der Verdampferkartusche ist dadurch gekennzeichnet, dass sie eine Tankhülle zur Bildung des Hauptvolumens des Vorratstanks, einen einen Aufnahmeraum bildenden Adapter zur Bildung des Nebenvolumens des Vorratstanks als Zwischenspeicher innerhalb des Hauptvolumens, sowie ein den Luftströmungskanal oder Teile davon bildendes Kanalelement als Hohlkörper umfasst. Tankhülle, Adapter und Kanalelement können einstückig hergestellt und gebildet sein, beispielsweise im Spritzgussverfahren oder dergleichen. Eine einteilige Ausführung ermöglicht eine besonders einfache und kostengünstige Fertigung. Die genannten Komponenten können aber auch separat ausgebildet sein, wobei die Komponenten dann z.B. mittels Presspassungen miteinander verbunden sind.

Vorteilhafterweise umfasst die Verdampferkartusche ein Trägerelement, das zum einen einen Durchgangskanal zur Bildung des Luftströmungskanals oder Teilen davon und zum anderen eine Ausnehmung zur Aufnahme der Verdampfereinheit aufweist, wobei das Trägerelement und die Verdampfereinheit eine bauliche Einheit bilden, die innerhalb des Aufnahmeraums des Adapters dichtend gegenüber der Umgebung angeordnet ist. Diese vorzugsweise vorgefertigte bauliche Einheit ist als eine Art Plug-in-Element leicht zu montieren, wobei die bauliche Einheit mit dem Adapter insbesondere vor der Befüllung des Vorratstanks mit der Flüssigkeit in die Tankhülle einsetzbar ist. Dadurch ist der Vorratstank schon vor dem Befüllen mindestens teilweise geschlossen, so dass ein Schutz gegenüber Verunreinigungen im Vorratstank existiert.

Vorzugsweise ist das den Zwischenspeicher bildende Nebenvolumen durch die vom Hauptvolumen des Vorratstanks abgewandte Innenfläche des Aufnahmeraumes des Adapters und die Außenfläche der aus Verdampfereinheit und Trägerelement gebildeten baulichen Einheit begrenzt, wobei die Innenfläche des Aufnahmeraumes des Adapters zur Bildung des Nebenvolumens mindestens teilweise beabstandet zur Außenfläche der baulichen Einheit ausgebildet und eingerichtet ist. Dadurch ist ein Zwischenraum gebildet, in dem die Flüssigkeit bevorratet werden kann. Der Adapter kann z.B. eine topfartige Ausgestaltung aufweisen, wobei sich die bauliche Einheit im Inneren des Adapters befindet, so dass das Dochtorgan permanent und direkt mit der im Zwischenspeicher befindlichen Flüssigkeit in Kontakt steht.

In einer bevorzugten Ausführungsform bilden das Trägerelement und das Kanalelement einen durch die gesamte Verdampferkartusche durchgängigen Schlot mit dem Luftströmungskanal, wobei das Trägerelement und das Kanalelement dichtend miteinander verbunden sind. Die dichtende Verbindung kann z.B. durch eine einstückige Ausbildung hergestellt sein. Vorzugsweise sind das Trägerelement und das Kanalelement jedoch separate Komponenten, die in- oder aneinandergesteckt sind. Ein kanalförmiger Abschnitt des Trägerelementes ist in das Kanalelement gesteckt, wobei zwischen dem kanalförmigen Abschnitt des Trägerelementes und dem Kanalelement ein Dichtungselement angeordnet ist. Optional kann der kanalförmige Abschnitt des Trägerelementes auch auf das Kanalelement gesteckt oder anderweitig dichtend mit diesem verbunden sein.

Besonders bevorzugt ist ausgehend vom Nebenvolumen mindestens eine Steigleitung ausgebildet und angeordnet, die zur Bildung einer Flüssigkeitsverbindung in das Hauptvolumen ragt. Mittels einer Steigleitung kann eine Flüssigkeitsverbindung zwischen dem Hauptvolumen und dem Nebenvolumen hergestellt sein. Die Steigleitung kann die einzige Flüssigkeitsverbindung darstellen. Die Steigleitung kann aber auch ergänzend zu anderen Öffnungen, Bohrungen, Spalten etc. die Flüssigkeitsverbindung optimieren, so dass der Zwischenspeicher zuverlässig mit Flüssigkeit aus dem Hauptvolumen versorgt wird.

Zweckmäßigerweise ist ein freies Ende der mindestens einen Strömungskanal aufweisenden Steigleitung zur Aufnahme von Flüssigkeit aus dem Hauptvolumen beabstandet zu der das Hauptvolumen begrenzenden Tankhülle des Vorratstanks ausgebildet. Durch den Abstand des freien Endes der Steigleitung zur Tankhülle bzw. zu anderen Begrenzungen des Vorratstanks wird ein Spalt bzw. ein Zugang zum Befüllen der Steigleitung geschaffen. Es können auch mehrere Steigleitungen vorgesehen sein, die in unterschiedlichen Bereichen in den Zwischenspeicher münden. Jede Steigleitung kann auch mehr als einen Strömungskanal aufweisen, um die Zuführmenge von Flüssigkeit aus dem Hauptvolumen in das Nebenvolumen konstant hoch zu halten.

Besonders bevorzugt ist innerhalb des Strömungskanals der Steigleitung ein Ventilelement angeordnet, das einerseits den Zufluss von Flüssigkeit aus dem Hauptvolumen in das Nebenvolumen ermöglicht und andererseits einen Abfluss von Flüssigkeit aus dem Nebenvolumen in das Hauptvolumen zumindest erschwert oder ganz verhindert. Beispielsweise kann im Strömungskanal ein Kugelventil angeordnet sein, dass z.B. alleine durch Schwerkraft oder mittels eines Federelementes die Kugel in der Schließstellung hält, um einen Rückfluss von Flüssigkeit aus dem Nebenvolumen in das Hauptvolumen zu verhindern, wobei die Kugel durch Schwerkraft und/oder die aus dem Hauptvolumen strömende Flüssigkeit aus der Schließstellung gedrückt wird, so dass Flüssigkeit aus dem Hauptvolumen in das Nebenvolumen bzw. in den Zwischenspeicher fließen kann.

Vorteilhafterweise ist jede Steigleitung im Wesentlichen parallel zum Luftströmungskanal ausgerichtet. Mit dieser Ausrichtung ist eine besonders effektive Zuführung von Flüssigkeit in den Zwischenspeicher gewährleistet.

Eine vorteilhafte Weiterbildung der Verdampferkartusche ist dadurch gekennzeichnet, dass die rohrförmige Tankhülle eine mundstückseitige Stirnseite und eine bodenseitige Stirnseite aufweist, wobei beide Stirnseiten durch ein Deckelelement dichtend verschlossen sind, wobei mindestens ein Deckelelement zum Befüllen des Vorratstanks lösbar an der Tankhülle befestigt ist. Dadurch ist einerseits eine Vormontage der Verdampferkartusche und andererseits eine einfache Befüllung des Vorratstanks mit Flüssigkeit gewährleistet. Die dichtende Verbindung von Deckelelement und Tankhülle kann durch eine einstückige Ausbildung/Herstellung oder durch Dichtelemente, wie z.B. einfache O-Ringe oder dergleichen erreicht werden.

Besonders bevorzugt ist das bodenseitig angeordnete Deckelelement lösbar mit der Tankhülle verbunden. Mit dieser Ausbildung ist eine bodenseitige Befüllung des Vorratstanks mit Flüssigkeit besonders einfach realisierbar. Als Deckelelemente können separate Deckel eingesetzt werden. In anderen Ausführungsformen kann das Deckelelement auch durch flanschartige Abschnitte von Trägerelement und/oder Adapter gebildet werden. Letztlich können aber auch das mundstückseitige Deckelelement oder sowohl das bodenseitige als auch das mundstückseitige Deckelelement lösbar an der Tankhülle befestigt sein.

Vorteilhafterweise ist das mundstückseitige Deckelelement einstückig mit der Tankhülle ausgebildet, wobei das mundstückseitige Deckelelement als Mundstück geformt ist. Dadurch kann auf Spalte, Verbindungsnähte, Klebestellen oder dergleichen verzichtet werden, was insbesondere vor dem Hintergrund, dass das Mundstück von der die Verdampferkartusche benutzenden Person zum Saugen in den Mund genommen wird, verbesserte Nutzungseigenschaften ermöglicht. Allerdings sind auch mehrstückige Ausbildungen, bei denen die Komponenten z.B. geklebt oder gelasert werden, grundsätzlich möglich.

Besonders bevorzugt weist der Adapter Mikrobohrungen und/oder Mikroöffnungen auf und/oder bildet diese zusammen mit der Tankhülle und/oder dem Kanalelement und/oder den Deckelelementen aus, durch die Flüssigkeit aus dem Hauptvolumen in das Nebenvolumen förderbar ist. Damit wird eine optimierte Zuführung der Flüssigkeit in das Nebenvolumen gewährleistet. Insbesondere Mikroöffnungen/Mikrospalte etc. entstehen ohne zusätzlichen Aufwand bei der Fertigung/Herstellung der einzelnen Komponenten. Im Gegenteil kann sogar kostengünstiger gefertigt werden, da die Komponenten mit größeren Toleranzen - und damit kostengünstiger - gefertigt werden können, die dann zu den Spaltmaßen führen, durch die Flüssigkeit in das Nebenvolumen fließen/kriechen kann.

Vorteilhafterweise ist das Nebenvolumen mindestens teilweise mit einem Flüssigkeit speichernden Material, vorzugsweise mit granulatartigen Körnern, gefüllt. Dadurch wird das Speichervolumen in unmittelbarer Nähe zum Dochtorgan optimiert. Letztlich bildet das mit Vliesmaterial und/oder Schaummaterial und/oder Fasermaterial und/oder den granulatartigen Körnern und/oder anderen Flüssigkeit speichernden Materialien oder Materialmischungen teilweise oder vollständig befüllte Nebenvolumen eine Art Erweiterung/Vergrößerung des Dochtorgans, wodurch eine größere Unabhängigkeit bei der Versorgung des Dochtorgans durch das Hauptvolumen erreicht wird, und zwar ohne die Befüllbarkeit des Tankvolumens einzuschränken oder zu behindern.

Die Aufgabe wird auch durch einen Inhalator der eingangs gennannten Art dadurch gelöst, dass die Verdampferkartusche nach einem oder mehreren der Ansprüche 1 bis 17 ausgebildet und eingerichtet ist.

Die sich daraus ergebenden Vorteile wurden bereits im Zusammenhang mit der Verdampferkartusche beschrieben, weshalb zur Vermeidung von Wiederholungen auf die vorstehenden Ausführungen verwiesen wird.

Weitere zweckmäßige und/oder vorteilhafte Merkmale und Weiterbildungen zur Verdampferkartusche und zum Inhalator ergeben sich aus den Unteransprüchen und der Beschreibung. Besonders bevorzugte Ausführungsformen der Verdampferkartusche und des Inhalators werden anhand der beigefügten Zeichnung näher erläutert. In der Zeichnung zeigt:
- Fig. 1: eine schematische Darstellung einer bevorzugten Ausführungsform eines erfindungsgemäßen Inhalators mit Kartuschenträger und Verdampferkartusche im Teilschnitt,
- Fig. 2: eine erste Ausführungsform einer erfindungsgemäßen Verdampferkartusche im Schnitt,
- Fig. 3: eine weitere Ausführungsform einer erfindungsgemäßen Verdampferkartusche,
- Fig. 4: eine weitere Ausführungsform einer erfindungsgemäßen Verdampferkartusche,
- Fig. 5: eine weitere Ausführungsform einer erfindungsgemäßen Verdampferkartusche,
- Fig. 6: eine weitere Ausführungsform eines Adapters für eine erfindungsgemäße Verdampferkartusche,
- Fig. 7: eine weitere Ausführungsform eines Adapters für eine erfindungsgemäße Verdampferkartusche, und
- Fig. 8: eine weitere Ausführungsform eines Adapters für eine erfindungsgemäße Verdampferkartusche.

Die in der Zeichnung dargestellte Verdampferkartusche dient als Bestandteil eines Inhalators zum Inhalieren von mit Wirkstoffen, z.B. Nikotin, angereichertem Dampf und/oder Aerosolen aus Flüssigkeiten, und ist entsprechend im Zusammenhang mit einer E-Zigarette beschrieben. Die Verdampferkartusche und der Inhalator sind in gleicher Weise zum Inhalieren von mit medizinischen Wirkstoffen angereichertem Dampf aus pharmazeutischen und/oder nahrungsergänzenden Produkten einsetzbar.

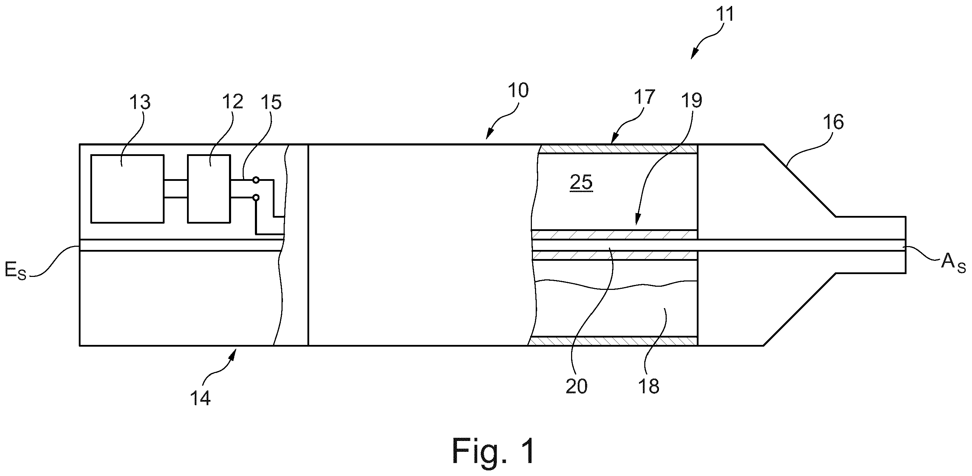

Die dargestellte Verdampferkartusche 10 bildet einen Bestandteil eines Inhalators 11. Dazu ist die Verdampferkartusche 10 zum mechanischen und elektrischen Verbinden mit einem mindestens eine elektronische Steuereinheit 12 und eine Energiequelle 13 umfassenden Kartuschenträger 14 ausgebildet und eingerichtet, wobei elektrische Kontakte 15 von der Verdampferkartusche 10 zur elektrischen Kontaktierung mit der Energiequelle 13 zum Kartuschenträger 14 führen (siehe insbesondere Figur 1). Der Inhalator 11 kann z.B. durch eine inhalierende Person aktiviert werden, beispielsweise als E-Zigarette, indem die Person an einem Mundstück 16 saugt, oder z.B. durch eine Pumpe aktiviert werden, z.B. als medizinisches Instrument für den Fall, dass die Person selbst nicht mehr oder nicht ausreichend saugen kann.

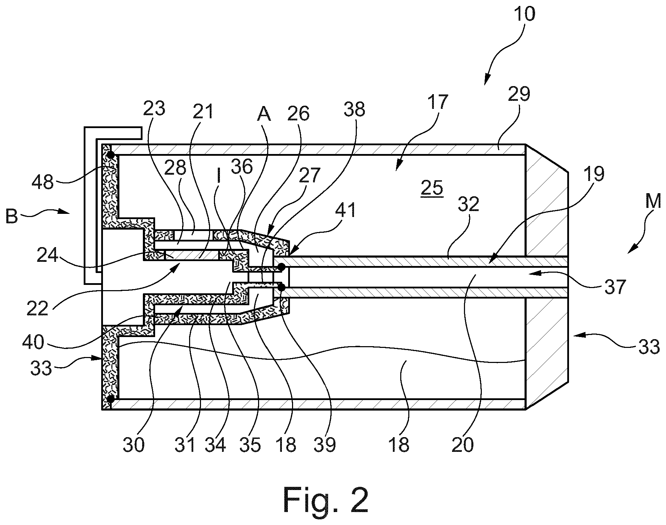

Die in den Figuren 2 bis 5 dargestellte Verdampferkartusche 10 als Bestandteil eines Inhalators 11 umfasst mindestens einen Vorratstank 17 zum Aufnehmen und Bevorraten einer Flüssigkeit 18 und mindestens einen Hohlkörper 19 mit einem durchgängigen Luftströmungskanal 20, wobei der Vorratstank 17 mindestens eine Zugangsöffnung 21 zum Luftströmungskanal 20 aufweist und im Bereich jeder Zugangsöffnung 21 eine sich über die gesamte Zugangsöffnung 21 erstreckende Verdampfereinheit 22 angeordnet ist, die ein dem Vorratstank 17 zugewandtes Dochtorgan 23 und ein dem Luftströmungskanal 20 zugewandtes Heizorgan 24 aufweist, wobei die Verdampfereinheit 22 flüssigkeitspermeabel ausgebildet ist, derart, dass Flüssigkeit 18 mindestens initial kapillar aus dem Vorratstank 17 durch die Verdampfereinheit 22 in Richtung des Luftströmungskanals 20 förderbar ist.

Der Hohlkörper 19 mit seinem mindestens einen Luftströmungskanal 20, zwei oder mehr Luftströmungskanäle 20 können ebenfalls vorgesehen sein, bildet einen Saugkanal/Schlot. Die Form des Hohlkörpers 19 kann ebenso wie der Verlauf des Luftströmungskanals 20 nahezu beliebig sein. Entscheidend ist, dass eine Eintrittsseite Es jedes Luftströmungskanals 20 offen zur Umgebung ist, um z.B. Luft ansaugen zu können, und dass eine Austrittsseite A_{S} offen ist, um z.B. einen Unterdruck erzeugen zu können, insbesondere durch das Saugen einer konsumierenden Person. Offen bedeutet in diesem Zusammenhang, dass die Eintrittsseite E_{S} und die Austrittsseite A_{S} luftdurchlässig sind. Im Bereich der Zugangsöffnung 21 zwischen Vorratstank 17 und Luftströmungskanal 20 bildet die Verdampfereinheit 22 eine Art Flüssigkeitssperre, die verhindert, dass Flüssigkeit 18 aus dem Vorratstank 17 direkt und als Flüssigkeit 18 in den Luftströmungskanal 20 fließt. Unabhängig von der Form und Ausbildung des Vorratstanks 17, es können auch zwei oder mehr Vorratstanks 17 vorgesehen sein, und des Hohlkörpers 19 sowie der Anordnung/Positionierung von Vorratstank 17 zu Hohlkörper 19 stellt die Verdampfereinheit 22 sicher, dass Flüssigkeit 18 zwangsläufig aus dem Vorratstank 17 in Richtung des Luftströmungskanals 20 geführt wird und spätestens beim Austritt aus der Verdampfereinheit 22 als Gas bzw. Dampf in den Luftströmungskanal 20 abgegeben wird.

Diese Verdampferkartusche 10 zeichnet sich erfindungsgemäß dadurch aus, dass der Vorratstank 17 im Inneren konstruktiv in ein Hauptvolumen 25 und mindestens ein Nebenvolumen 26 unterteilt ist, wobei das Hauptvolumen 25 und das Nebenvolumen 26 miteinander flüssigkeitsgekoppelt sind und lediglich das Nebenvolumen 26 in direktem Kontakt zur Verdampfereinheit 22 steht, derart, dass Flüssigkeit 18 aus dem Hauptvolumen 25 ausschließlich und zwangsläufig über das Nebenvolumen 26 mit dem Dochtorgan 23 in Kontakt bringbar ist.

Beim Durchtritt durch die das Dochtorgan 23 und das Heizorgan 24 umfassende Verdampfereinheit 22 wird im Betrieb der Verdampferkartusche 10 aus der Flüssigkeit 18 des Vorratstanks 17 zum Luftströmungskanal 20 hin Dampf und/oder Aerosol gebildet, wobei die poröse und Mikrokanäle und/oder Mikroöffnungen aufweisende Struktur des Dochtorgans 23, das Dochtorgan 23 kann z.B. als aus mehreren miteinander verwobenen/verdrillten Fäden/Fasern z.B. aus Baumwolle oder Glasfasern gebildeter Faserdocht, als einstückiger und aus keramischen Werkstoffen bestehender Dochtblock, oder als aus granulatartigen Körnern gebildetes Dochtorgan ausgebildet sein, einerseits ein Speichermedium für Flüssigkeit 18 bildet und andererseits einen Strömungswiderstand darstellt. Die Strömungsrichtung der Flüssigkeit 18 erfolgt vom Vorratstank 17 durch die Verdampfereinheit 22 in Richtung des Luftströmungskanals 20.

Die erfindungsgemäße Verdampferkartusche 10 kann als Einwegartikel eine bauliche Einheit sein, die die Komponenten Vorratstank 17, Hohlkörper 19 und Verdampfereinheit 22 beinhaltet. Die Verdampferkartusche 10 kann aber auch mehrteilig ausgebildet sein, wobei sich Komponenten der Verdampferkartusche 10 auf den Einwegartikel und den Mehrwegartikel verteilen, derart, dass z.B. der Vorratstank 17 ein Einwegartikel ist, der erst beim Zusammenführen mit einem Kartuschenträger 14, der ein Mehrwegartikel sein kann und neben der elektronischen Steuereinheit 12 und der Energiequelle 13 auch Bestandteile der Verdampferkartusche 10, wie z.B. den Hohlkörper 19 und die Verdampfereinheit 22, umfassen kann, zur baulichen Einheit der Verdampferkartusche 10 führt. Die Verdampferkartusche 10 definiert sich entsprechend über ihre Komponenten, nämlich Vorratstank 17, Hohlkörper 19 mit Luftströmungskanal 20, und Verdampfereinheit 22, und nicht über die konstruktive/bauliche Zuordnung der Komponenten zum Mehrwegartikel bzw. Einwegartikel.

Die im Folgenden beschriebenen Merkmale und Weiterbildungen stellen für sich betrachtet oder in Kombination miteinander bevorzugte Ausführungsformen dar. Es wird ausdrücklich darauf hingewiesen, dass Merkmale, die in den Ansprüchen und/oder der Beschreibung und/oder der Zeichnung zusammengefasst oder in einer gemeinsamen Ausführungsform beschrieben sind, auch funktional eigenständig die weiter oben beschriebene Verdampferkartusche 10 weiterbilden können.

Wie in allen bevorzugten Ausführungsformen der Verdampferkartusche 10 gemäß den Figuren 2 bis 5 dargestellt, ist der Vorratstank 17 im Inneren durch eine Trennwand 27 oder dergleichen in das Hauptvolumen 25 und mindestens ein Nebenvolumen 26 unterteilt, wobei das Nebenvolumen 26 zur Aufnahme von Flüssigkeit 18 aus dem Hauptvolumen 25 mit diesem in Flüssigkeitsverbindung steht. Als Trennwand 27 dient bevorzugt ein topfförmiges Element, das einerseits mit dem Hohlkörper 19 verbunden ist und andererseits die Verdampfereinheit 22 mindestens radial vollständig umgibt. Als Trennwand 27 können aber auch konstruktiv anders ausgebildete Elemente oder Komponenten eingesetzt werden, die zum einen die direkte Zuführung von Flüssigkeit 18 aus dem Hauptvolumen 25 an die Verdampfereinheit 22 und konkret an das Dochtorgan 23 verhindern und zum anderen einen Aufnahmeraum zum Aufnehmen von Flüssigkeit 18 bilden. Das Nebenvolumen 26 bildet einen Zwischenspeicher für die Flüssigkeit 18 und stellt eine Flüssigkeitsverbindung zwischen dem Hauptvolumen 25 und dem Dochtorgan 23 der Verdampfereinheit 22 her. Der Zwischenspeicher schirmt das Dochtorgan 23 mit Ausnahme mindestens einer Zugangsöffnung 28 zur Herstellung der Flüssigkeitsverbindung zwischen dem Nebenvolumen 26 und dem Hauptvolumen 25 gegenüber dem Hauptvolumen 25 ab (siehe z.B. Figur 2). In einzelnen Ausführungsformen kann eine einzelne Zugangsöffnung 28 ausgebildet sein. Vorzugsweise sind jedoch mehrere Zugangsöffnungen 28 vorgesehen. Hierzu werden weiter unten noch detailliert weitere Beispiele beschrieben.

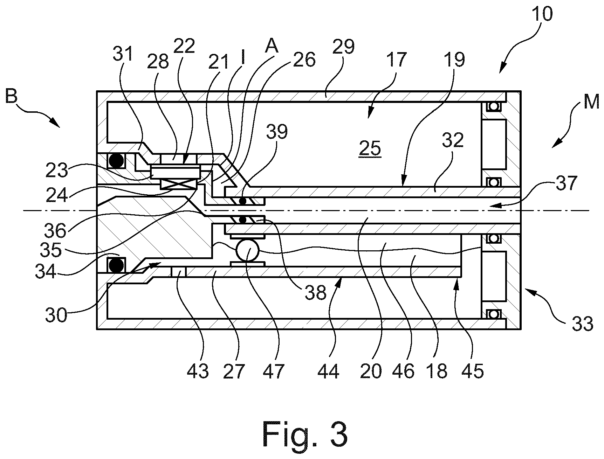

Optional und bevorzugt umfasst die Verdampferkartusche 10 eine Tankhülle 29 zur Bildung des Hauptvolumens 25 des Vorratstanks 17, einen einen Aufnahmeraum 30 bildenden Adapter 31 zur Bildung des Nebenvolumens 26 des Vorratstanks 17 als Zwischenspeicher innerhalb des Hauptvolumens 25, sowie ein den Luftströmungskanal 20 oder Teile davon bildendes Kanalelement 32 als Hohlkörper 19. Die Tankhülle 29 bildet besonders bevorzugt einen zylindrischen, rohrförmigen Körper, wobei die äußere und die innere Gestaltung des Körpers ebenso abweichend ausgebildet sein können wie dessen Querschnittsform. Der durch die Tankhülle 29 gebildete Körper weist zwei Stirnseiten auf, nämlich eine bodenseitige Stirnseite B und eine mundstückseitige Stirnseite M, die durch Deckelelemente 33 oder dergleichen dichtend verschlossen sind, so dass die Flüssigkeit 18 sicher innerhalb des Vorratstanks 17 bevorratet ist. Der Adapter 31 als Trennwand 27 zur konstruktiven Bildung des Zwischenspeichers kann als separate Komponente ausgebildet sein. In diesem Fall ist der Adapter 31 auf oder in das Kanalelement 32 gesteckt (siehe z.B. Figuren 2, 4 und 5). Der Adapter 31 kann aber auch einstückig mit dem Kanalelement 32 ausgebildet sein (siehe z.B. Figur 3). Der Adapter 31 verlängert das Kanalelement 32 in Richtung bodenseitiger Stirnwand B, was insbesondere bei der Befüllung des Vorratstanks 17 von der bodenseitigen Stirnwand B dazu führt, dass innerhalb des Vorratstanks 17 ein höherer Füllspiegel erreicht werden kann. Das Kanalelement 32 selbst, das sich durch den gesamten Vorratstank 17 erstrecken kann, erstreckt sich in den bevorzugten Ausführungsbeispielen ausgehend von der mundstückseitigen Stirnseite M nur durch einen Teil des Vorratstanks 17. Das Kanalelement 32, dessen Verlauf und Querschnitt variieren kann, ist beispielsweise ein einfaches Rohr.

Die Verdampferkartusche 10 umfasst vorzugsweise weiterhin ein Trägerelement 34, das zum einen einen Durchgangskanal 35 zur Bildung des Luftströmungskanals 20 oder Teilen davon und zum anderen eine Ausnehmung 36 zur Aufnahme der Verdampfereinheit 22 aufweist, wobei das Trägerelement 34 und die Verdampfereinheit 22 eine bauliche Einheit bilden, die innerhalb des Aufnahmeraums 30 des Adapters 31 dichtend gegenüber der Umgebung angeordnet ist. Diese Einheit, die auch als Inside-Verdampfer-Einheit bezeichnet wird, ist zum einen mindestens teilweise, in jedem Fall aber mit der gesamten Verdampfereinheit 22 innerhalb des Aufnahmeraums 30 des Adapters 31 angeordnet, und zum anderen mit dem Kanalelement 32 verbunden. Das Trägerelement 34 und das Kanalelement 32 bilden einen durch die gesamte Verdampferkartusche 10 durchgängigen Schlot 37 mit dem Luftströmungskanal 20, wobei das Trägerelement 34 und das Kanalelement 32 dichtend miteinander verbunden sind. Die dichtende Verbindung kann z.B. durch eine einstückige Ausbildung hergestellt sein. Optional sind das Trägerelement 34 und das Kanalelement 32 jedoch separate Komponenten, die in- oder aneinandergesteckt sind. Ein kanalförmiger Abschnitt 38 des Trägerelementes 34 ist in das Kanalelement 32 gesteckt, wobei zwischen dem kanalförmigen Abschnitt 38 des Trägerelementes 34 und dem Kanalelement 32 ein Dichtungselement 39 z.B. in Form eines einfachen O-Rings, angeordnet ist. Optional kann der kanalförmige Abschnitt 38 des Trägerelementes 34 auch auf das Kanalelement 32 gesteckt oder anderweitig mit diesem verbunden sein.

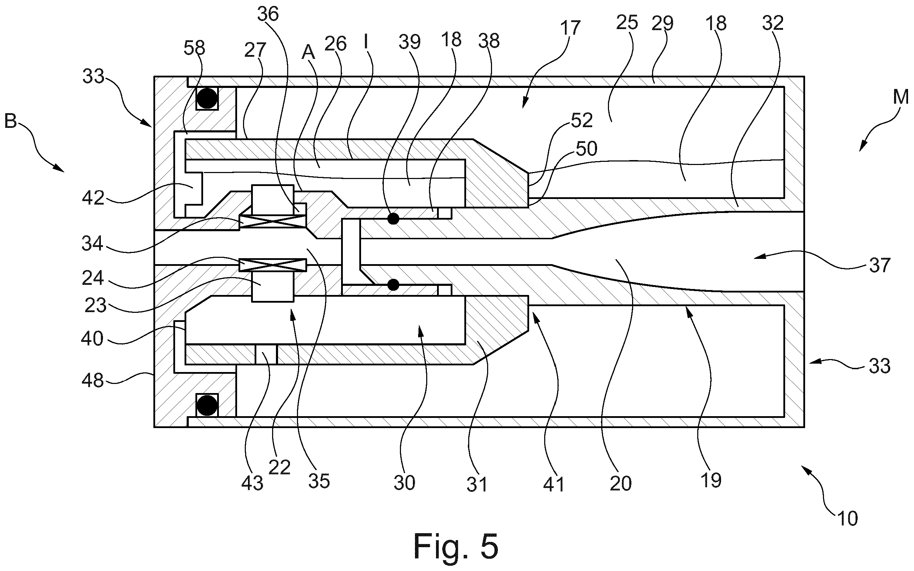

In den dargestellten Ausführungsformen ist das den Zwischenspeicher bildende Nebenvolumen 26 durch die vom Hauptvolumen 25 des Vorratstanks 17 abgewandte Innenfläche I des Aufnahmeraumes 30 des Adapters 31 und die Außenfläche A der aus Verdampfereinheit 22 und Trägerelement 34 gebildeten baulichen Einheit begrenzt, wobei die Innenfläche I des Aufnahmeraumes 30 des Adapters 31 zur Bildung des Nebenvolumens 26 mindestens teilweise beabstandet zur Außenfläche A der baulichen Einheit ausgebildet und eingerichtet ist. Durch die oder jede Zugangsöffnung 28 gelangt die Flüssigkeit 18 aus dem Hauptvolumen 25 in das Nebenvolumen 26 und wird darin zwischengespeichert. In der Ausführungsform gemäß Figur 2 ist eine Zugangsöffnung 28 in der Wandung des Adapters 31 ausgebildet. Zusätzlich dazu kann Flüssigkeit 18 auch noch durch Montagepassungen/Presspassungen in das Nebenvolumen 26 gelangen, beispielsweise im Kontaktbereich zwischen dem freien Randbereich 40 des Adapters 31 und dem Trägerelement 34 und/oder im Steckbereich 41 zwischen Adapter 31 und Kanalelement 32 (siehe hierzu z.B. Figur 2). In anderen Ausführungsformen (siehe hierzu z.B. Figur 5) kann im freien Randbereich 40, der beanstandet zu einem stirnseitigen Deckelelement 33 angeordnet sein kann, mindestens ein Zulauf 42 ausgebildet sein. Alternativ oder kumulativ können z.B. in der Umfangswandung des Adapter 31 auch Mikroöffnungen 43 oder dergleichen ausgebildet sein, die eine Zuführung von Flüssigkeit 18 in das Nebenvolumen 26 unterstützen.

Ebenfalls alternativ oder kumulativ ist in bevorzugten Ausführungsformen der Verdampferkartusche 10 ausgehend vom Nebenvolumen 26 mindestens eine Steigleitung 44 ausgebildet und angeordnet, die zur Bildung einer Flüssigkeitsverbindung in das Hauptvolumen 25 ragt (siehe z.B. Figur 3). Ein freies Ende 45 der mindestens einen Strömungskanal 46 aufweisenden Steigleitung 44 ist zur Aufnahme von Flüssigkeit 18 aus dem Hauptvolumen 25 beabstandet zu der das Hauptvolumen 25 begrenzenden Tankhülle 29 oder einem Deckelelement 33 des Vorratstanks 17 ausgebildet. Innerhalb der Steigleitung 44 können auch zwei oder mehr Strömungskanäle 46 ausgebildet sein. Es können auch zwei oder mehr Steigleitungen 44 angeordnet sein, die eine Flüssigkeitsverbindung zwischen Hauptvolumen 25 und Nebenvolumen 26 herstellen. Optional ist innerhalb des Strömungskanals 46 der Steigleitung 44 ein Ventilelement 47 oder dergleichen angeordnet, das einerseits den Zufluss von Flüssigkeit 18 aus dem Hauptvolumen 25 in das Nebenvolumen 26 ermöglicht und andererseits einen Abfluss von Flüssigkeit 18 aus dem Nebenvolumen 26 in das Hauptvolumen 25 zumindest erschwert. Der Verlauf und/oder die Ausrichtung der Steigleitung 44 bzw. des darin ausgebildeten Strömungskanals 46 kann variieren. Vorzugsweise ist jede Steigleitung 44 bzw. der Strömungskanal 46 im Wesentlichen parallel zum Luftströmungskanal 20 ausgerichtet.

Bei der Ausführungsform gemäß Figur 2 sind die Tankhülle 29 und das mundstückseitige Deckelelement 33 einstückig ausgebildet. Der Adapter 31, das Kanalelement 32 und das Trägerelement 34 sind separate Komponenten. Der Adapter 31 ist unter Bildung einer Montagepassung/Presspassung auf ein freies, in Richtung bodenseitiger Stirnseite B weisenden Ende des Kanalelementes 32 gesteckt und befestigt. Das Trägerelement 34 mit der Verdampfereinheit 22 ist abschnittsweise, nämlich mit dem die Verdampfereinheit 22 aufweisenden Abschnitt, im Aufnahmeraum 30 des Adapters 31 positioniert, derart, dass das Dochtorgan 23 im Bereich der Zugangsöffnung 28 liegt. Der kanalförmige Abschnitt 38 des Trägerelementes 34 ist dichtend, z.B. mittels eines einfachen O-Rings, mit dem Kanalelement 32 verbunden und daran befestigt. Das Trägerelement 34 umfasst des Weiteren einen flanschartigen Kragen 48, so dass das Trägerelement 34 das lösbare Deckelelement 33 auf der bodenseitigen Stirnseite B bildet. Der flanschartige Kragen 48 des Trägerelementes 34 ist dichtend, z.B. mittels eines einfachen O-Rings, mit der Tankhülle 29 verbunden. Dem Heizorgan 24 sind elektrische Kontakte 15 zur Anbindung an die Energiequelle 13 zugeordnet.

Die Ausführungsform gemäß Figur 3 zeigt eine Verdampferkartusche 10, bei der die Tankhülle 29, der Adapter 31, das Kanalelement 32 und die Steigleitung 44 einstückig ausgebildet und gefertigt sind, beispielsweise im Spritzgussverfahren. Das Trägerelement 34 mit der Verdampfereinheit 22 ist vollständig innerhalb des Aufnahmeraums 30 des Adapters 31, der zur bodenseitigen Stirnwand B offen ausgebildet ist, angeordnet, derart, dass das Dochtorgan 23 im Bereich der Zugangsöffnung 28 positioniert ist. Der kanalförmige Abschnitt 38 des Trägerelementes 34 ist dichtend, z.B. mittels eines einfachen O-Rings, mit dem Kanalelement 32 verbunden und daran befestigt. Das Trägerelement 34 ist weiterhin zur Umgebung dichtend, z.B. mittels eines einfachen O-Rings, gegenüber der Innenfläche I des Aufnahmeraums 30 ausgebildet und bildet damit quasi das Deckelelement 33 an der bodenseitigen Stirnseite B. An der mundstückseitigen Stirnseite M ist der Vorratstank 17 durch ein lösbares Deckelelement 33 geschlossen, wobei das Deckelelement 33 sowohl zur Tankhülle 29 als auch zum Kanalelement 32 dichtend, z.B. mittels einfacher O-Ringe, ausgebildet ist.

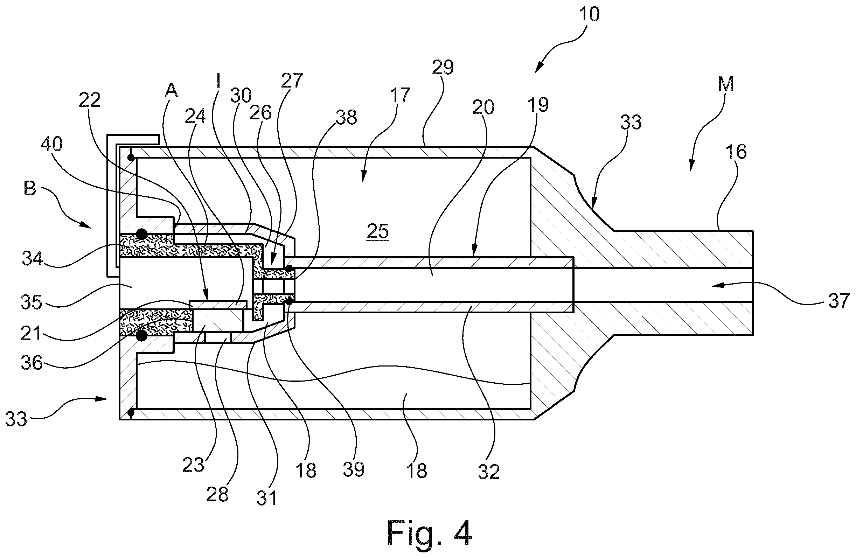

Bei der Verdampferkartusche 10 gemäß Figur 4 sind die Tankhülle 29 und das mundstückseitige Deckelelement 33 einstückig ausgebildet. Der Adapter 31, das Kanalelement 32 und das Trägerelement 34 sind separate Komponenten. Der Adapter 31 ist unter Bildung einer Montagepassung/Presspassung auf ein freies, in Richtung bodenseitiger Stirnseite B weisenden Ende des Kanalelementes 32 gesteckt und befestigt. Das Trägerelement 34 mit der Verdampfereinheit 22 ist abschnittsweise, nämlich mit dem die Verdampfereinheit 22 aufweisenden Abschnitt, in den Aufnahmeraum 30 des Adapters 31 positioniert, derart, dass das Dochtorgan 23 im Bereich der Zugangsöffnung 28 liegt. Der kanalförmige Abschnitt 38 des Trägerelementes 34 ist dichtend, z.B. mittels eines einfachen O-Rings, mit dem Kanalelement 32 verbunden und daran befestigt. Der Vorratstank 17 ist auf der bodenseitigen Stirnseite B mit einem lösbar befestigen Deckelelement 33 verschlossen, wobei das Deckelelement 33 zum einen dichtend, beispielsweise mittels eines einfachen O-Rings, gegenüber dem Trägerelement 34 ausgebildet ist, und zum anderen dichtend, beispielsweise mittels eines einfachen O-Rings, gegenüber der Tankhülle 29 ausgebildet ist. In der Ausführung gemäß Figur 4 ist das mundstückseitige Deckelelement 33 nicht nur einstückig mit der Tankhülle 29 ausgebildet, sondern das mundstückseitige Deckelelement 33 ist auch als Mundstück 16 geformt zum Saugen durch eine Person.

Bei der Verdampferkartusche 10 gemäß Figur 5 sind die Tankhülle 29 und das mundstückseitige Deckelelement 33 ebenfalls einstückig ausgebildet, wobei zusätzlich das Kanalelement 32 einstückig mit der Tankhülle 29 und dem Deckelelement 33 ausgebildet ist. Der Adapter 31 und das Trägerelement 34 sind separate Komponenten. Der Adapter 31 ist gegen einen Absatz 50 an der Außenfläche des Kanalelementes 32 aufgesetzt, wobei der Absatz 50 die Position für den Adapter 31 bestimmt. Das Trägerelement 34 mit der Verdampfereinheit 22 ist abschnittsweise, nämlich mit dem die Verdampfereinheit 22 aufweisenden Abschnitt, im Aufnahmeraum 30 des Adapters 31 positioniert. Das Dochtorgan 23 ist radial umlaufend um das Trägerelement 34 ausgebildet, so dass das Dochtorgan 23 in jeder Orientierung der Verdampferkartusche 10 mindestens mit einem Abschnitt an der tiefsten Position des Nebenvolumens 26 liegt. Es kann ein Heizorgan 24 vorgesehen sein. Optional können z.B. auch zwei Heizorgane 24 vorgesehen sein. Das Heizorgan 24 kann auch umlaufend ausgebildet und angeordnet sein. Als Zugangsöffnung 28 ist der Überlauf 42 ausgebildet. Zusätzlich sind in der Umfangsfläche des Adapters 31 eine oder mehrere Mikroöffnungen 43 ausgebildet. Der kanalförmige Abschnitt 38 des Trägerelementes 34 ist dichtend, z.B. mittels eines einfachen O-Rings, mit dem Kanalelement 32 verbunden und daran befestigt. Der Vorratstank 17 ist auf der bodenseitigen Stirnseite B mit einem lösbar befestigen Deckelelement 33 verschlossen, wobei das Deckelelement 33 durch den flanschartigen Kragen 48 des Trägerelementes 34 gebildet ist. Der flanschartige Kragen 48 des Trägerelementes 34 ist dichtend, z.B. mittels eines einfachen O-Rings, mit der Tankhülle 29 verbunden. Der freie Randbereich 40 des Adapters 31 ist zum einen beabstandet zum Deckelelement 33 ausgebildet. Zum anderen ist zumindest ein Spalt 58 zwischen der radial gerichteten Außenfläche des Adapters 31 und dem Deckelelement 33 gebildet, um einen Zugang für die Flüssigkeit 18 in das Nebenvolumen 26 zu schaffen.

Das Nebenvolumen 26 kann mindestens teilweise mit einem Flüssigkeit speichernden Material, vorzugsweise mit granulatartigen Körnern, gefüllt sein. Vorzugsweise ist das gesamte Nebenvolumen 26 z.B. durch Vliesmaterial oder Schaummaterial oder Fasermaterial oder das Dochtorgan 23 erweiternde, granulatartige Körner aufgefüllt. Anstelle der durchgängig beschriebenen O-Ringe als Dichtelement können auch andere übliche Dichtungen eingesetzt werden. Die dargestellten Ausführungsformen, die keine Steigleitung 44 aufweisen, können selbstverständlich durch solche ergänzt werden.

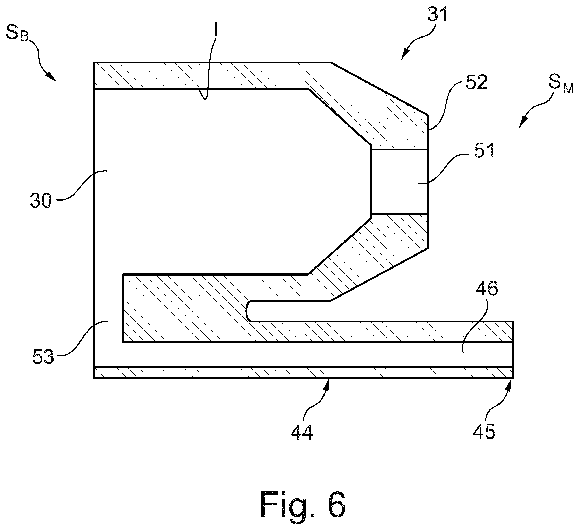

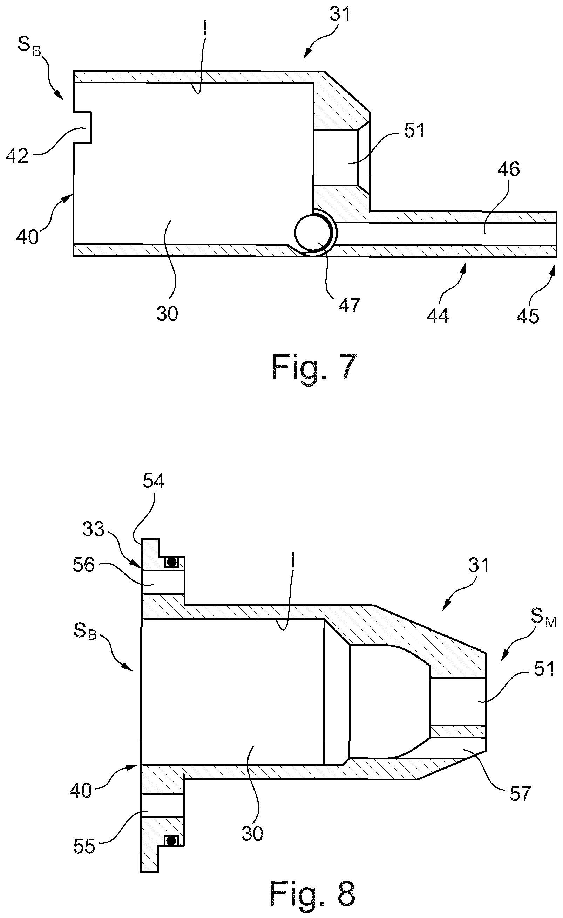

In den Figuren 6 bis 8 sind weitere Ausgestaltungen des Adapters 31 dargestellt, die in alle vorgenannten Verdampferkartuschen 10 integrierbar sind. Der Adapter 31 gemäß Figur 6 ist einstückig mit einer Steigleitung 44 ausgebildet. Der Adapter 31 weist auf der mundstückseitigen Seite S_{M} eine Durchgangsöffnung 51 auf, in die das Kanalelement 32 einsetzbar ist. Die Stirnseite selbst bildet einen Anschlag 52 für die Montageposition am Kanalelement 32. Auf der bodenseitigen Seite S_{B} ist ein Materialreduzierung 53 ausgebildet, die einen Zulauf/Überlauf für die Flüssigkeit 18 aus der Steigleitung 44 in den Aufnahmeraum 30 bildet. Der Adapter 31 gemäß Figur 7 ist ähnlich ausgebildet. Allerdings ist am freien Randbereich 40 des Adapters 31 der Zulauf 42 ausgebildet. In der Steigleitung 44 ist im Übergangsbereich zum Aufnahmeraum das Ventilelement 47 angeordnet. Der Adapter 31 gemäß Figur 8 weist einen flanschartigen Abschnitt 54 auf, derart, dass dieser Abschnitt 54 das bodenseitige Deckelelement 33 bildet, das lösbar und dichtend, z.B. mittels eines einfachen O-Rings, mit der Tankhülle 29 verbindbar ist. In dem flanschartigen Abschnitt 54 sind eine verschließbare Befüllöffnung 55 und eine verschließbare Entlüftungsöffnung 56 ausgebildet. Zusätzlich weist der Adapter 31 auf der mundstückseitigen Seite S_{M} neben der Durchgangsöffnung 51 zur Aufnahme des Kanalelementes 32 eine Durchgangsöffnung 57 zur Aufnahme einer Steigleitung 44 auf. Der Vorteil des Adapters 31 gemäß Figur 8 liegt darin, dass die gesamte Verdampferkartusche 10 vormontiert werden kann und einen nahezu vollständig geschlossenen Vorratstank 17 aufweist, wenn die Flüssigkeit 18 über die Befüllöffnung 55 befüllt wird, so dass die Gefahr von Verunreinigungen der Flüssigkeit 18 im Vorratstank 17 deutlich reduziert ist.

Die Montage einer Verdampferkartusche 10, bei der die Flüssigkeit 18 von der bodenseitigen Stirnseite B in den Vorratstank 17 befüllt wird, wird anhand der Figur 5 beispielhaft beschrieben. Zunächst wird die Tankhülle 29 mit dem Kanalelement 32 einstückig hergestellt und bereitgestellt. Anschließend wird der Adapter 31 in das bodenseitige, offene Ende des Vorratstanks 17 eingeführt und mittels des Anschlags 52, der gegen den Absatz 50 stößt, positioniert und dann befestigt. Durch die Positionierung wird die Spaltbildung im freien Randbereich 40 des Adapters 31 sichergestellt. Dann wird die Flüssigkeit 18 in den Vorratstank gefüllt, und zwar maximal bis zur Oberkante des freien Randbereiches 40. Durch den Adapter 31 kann die Füllhöhe der Flüssigkeit 18 signifikant erhöht werden, da der Adapter 31 das Kanalelement 32 quasi in Richtung der bodenseitigen Stirnseite B verlängert. Anschließend wird das Trägerelement 34 mit der Verdampfereinheit 22 als Einheit montiert, wobei der flanschartige Kragen 48 des Trägerelementes 34 als Deckelelement 33 dient und den Vorratstank 17 verschließt. Weist die Verdampferkartusche 10 einen Adapter 31 gemäß Figur 8 auf, wird zunächst die gesamte Verdampferkartusche 10 montiert, bevor die Flüssigkeit 18 zugeführt wird.

Die Montage einer Verdampferkartusche 10, bei der die Flüssigkeit 18 von der mundstückseitigen Stirnseite M befüllt wird, wird anhand der Figur 3 beispielhaft beschrieben. Zunächst wird das die Tankhülle 29, den Adapter 31, das Kanalelement 32 und die Steigleitung 44 umfassende Bauteil einstückig hergestellt und bereitgestellt. Anschließend wird die aus Trägerelement 34 und Verdampfereinheit 22 gebildete Einheit in dem Adapter 31 montiert. Danach wird der Vorratstank 17 über die mundstückseitige Stirnseite M mit der Flüssigkeit 18 befüllt. Nach dem Befüllen wird die mundstückseitige Stirnseite M mittels des Deckelelementes 33 dichtend verschlossen.

Das Funktionsprinzip des erfindungsgemäßen Inhalators 11, der eine Verdampferkartusche 10 gemäß der Erfindung umfasst, wird beispielhaft anhand einer E-Zigarette als Inhalator 11 insbesondere mit Bezug auf die Figur 1 beschrieben. Eine konsumierende 17 der Verdampferkartusche 10 eine Flüssigkeit 18 befindet, die beispielsweise Glycerin, Propylenglycol und ggf. weitere Wirkstoffe und/oder Geschmacksstoffe enthält. Durch das Saugen wird in dem Luftströmungskanal 20 ein Unterdruck erzeugt, der seinerseits z.B. über einen nicht dargestellten Sensor die Steuereinheit 12 aktiviert. Die Steuereinheit 12 steuert das Heizorgan 24, das von der Energiequelle 13 mit Energie versorgt wird. Flüssigkeit 18 aus dem Vorratstank 17 wird mittels des Dochtorgans 23 mindestens initial kapillar durch Mikrokanäle aus dem Vorratstank 17, nämlich aus dem Hauptvolumen 25 über das Nebenvolumen 26 vom Dochtorgan 23 in Richtung des Heizorganes 24 transportiert. Am bzw. im erwärmten Heizorgan 24 wird die Flüssigkeit 18 zu Gas bzw. Dampf umgewandelt, wobei das Heizorgan 24 die Flüssigkeit 18 bzw. das daraus gebildete Gas bzw. den daraus gebildeten Dampf aufgrund der flüssigkeits- und gas- bzw. dampfpermeablen Struktur in Richtung des Luftströmungskanals 20 transportiert und an diesen abgibt. Das aus dem Heizorgan 24 austretende Gas vermischt sich in dem Luftströmungskanal 20 mit dem Luftstrom, wobei es zu dem eigentlichen Rekondensations-/Dampfbildungsvorgang kommt, und wird von der konsumierenden Person angesaugt und inhaliert.

Durch das Nebenvolumen 26 ist selbst bei sich leerendem bzw. entleertem Hauptvolumen 25 noch ein Rest von Flüssigkeit 18 zwischengespeichert, und zwar in unmittelbarer Nähe und in Kontakt mit dem Dochtorgan 23, so dass eine weitere kontinuierliche Versorgung des Dochtorgans 23 mit Flüssigkeit 18 zumindest für einige Inhalationszüge bzw. Verdampfungsvorgänge sichergestellt ist.

## Patentansprüche

1. Verdampferkartusche (10) geeignet für ein Inhalator (11), umfassend mindestens einen Vorratstank (17) zum Aufnehmen und Bevorraten einer Flüssigkeit (18) und mindestens einen Hohlkörper (19) mit einem durchgängigen Luftströmungskanal (20), wobei der Vorratstank (17) mindestens eine Zugangsöffnung (21) zum Luftströmungskanal (20) aufweist und im Bereich jeder Zugangsöffnung (21) eine sich über die gesamte Zugangsöffnung (21) erstreckende Verdampfereinheit (22) angeordnet ist, die ein dem Vorratstank (17) zugewandtes Dochtorgan (23) und ein dem Luftströmungskanal (20) zugewandtes Heizorgan (24) aufweist, wobei die Verdampfereinheit (22) flüssigkeitspermeabel ausgebildet ist, derart, dass Flüssigkeit (18) mindestens initial kapillar aus dem Vorratstank (17) durch die Verdampfereinheit (22) in Richtung des Luftströmungskanals (20) förderbar ist, **dadurch gekennzeichnet, dass** der Vorratstank (17) im Inneren konstruktiv in ein Hauptvolumen (25) und mindestens ein Nebenvolumen (26) unterteilt ist, wobei das Hauptvolumen (25) und das Nebenvolumen (26) miteinander flüssigkeitsgekoppelt sind und lediglich das Nebenvolumen (26) in direktem Kontakt zur Verdampfereinheit (22) steht, derart, dass Flüssigkeit (18) aus dem Hauptvolumen (25) ausschließlich und zwangsläufig über das Nebenvolumen (26) mit dem Dochtorgan (23) in Kontakt bringbar ist, wobei der Vorratstank (17) im Inneren durch eine Trennwand (27) oder dergleichen in das Hauptvolumen (25) und mindestens ein Nebenvolumen (26) unterteilt ist, wobei das Nebenvolumen (26) zur Aufnahme von Flüssigkeit (18) aus dem Hauptvolumen (25) mit diesem in Flüssigkeitsverbindung steht.

2. Verdampferkartusche (10) nach Anspruch 1, **dadurch gekennzeichnet, dass** das Nebenvolumen (26) einen Zwischenspeicher bildet und eine Flüssigkeitsverbindung zwischen dem Hauptvolumen (25) und dem Dochtorgan (23) der Verdampfereinheit (22) herstellt.

3. Verdampferkartusche (10) nach Anspruch 2, **dadurch gekennzeichnet, dass** der Zwischenspeicher das Dochtorgan (23) mit Ausnahme mindestens einer Zugangsöffnung (28) zur Herstellung der Flüssigkeitsverbindung zwischen dem Nebenvolumen (26) und dem Hauptvolumen (25) gegenüber dem Hauptvolumen (25) abschirmt.

4. Verdampferkartusche (10) nach einem oder mehreren der Ansprüche 1 bis 3, **dadurch gekennzeichnet, dass** sie eine Tankhülle (29) zur Bildung des Hauptvolumens (25) des Vorratstanks (17), einen einen Aufnahmeraum (30) bildenden Adapter (31) zur Bildung des Nebenvolumens (26) des Vorratstanks (17) als Zwischenspeicher innerhalb des Hauptvolumens (25), sowie ein den Luftströmungskanal (20) oder Teile davon bildendes Kanalelement (32) als Hohlkörper (19) umfasst.

5. Verdampferkartusche (10) nach Anspruch 4, **dadurch gekennzeichnet, dass** sie ein Trägerelement (34) umfasst, das zum einen einen Durchgangskanal (35) zur Bildung des Luftströmungskanals (20) oder Teilen davon und zum anderen eine Ausnehmung (36) zur Aufnahme der Verdampfereinheit (22) aufweist, wobei das Trägerelement (34) und die Verdampfereinheit (22) eine bauliche Einheit bilden, die innerhalb des Aufnahmeraums (30) des Adapters (31) dichtend gegenüber der Umgebung angeordnet ist.

6. Verdampferkartusche (10) nach Anspruch 5, **dadurch gekennzeichnet, dass** das den Zwischenspeicher bildende Nebenvolumen (26) durch die vom Hauptvolumen (25) des Vorratstanks (17) abgewandte Innenfläche (I) des Aufnahmeraumes (30) des Adapters (31) und die Außenfläche (A) der aus Verdampfereinheit (22) und Trägerelement (34) gebildeten baulichen Einheit begrenzt ist, wobei die Innenfläche (I) des Aufnahmeraumes (30) des Adapters (31) zur Bildung des Nebenvolumens (26) mindestens teilweise beabstandet zur Außenfläche (A) der baulichen Einheit ausgebildet und eingerichtet ist.

7. Verdampferkartusche (10) nach einem oder mehreren der Ansprüche 4 bis 6, **dadurch gekennzeichnet, dass** das Trägerelement (34) und das Kanalelement (32) einen durch die gesamte Verdampferkartusche (10) durchgängigen Schlot (37) mit dem Luftströmungskanal (20) bilden, wobei das Trägerelement (34) und das Kanalelement (32) dichtend miteinander verbunden sind.

8. Verdampferkartusche (10) nach einem oder mehreren der Ansprüche 4 bis 7, **dadurch gekennzeichnet, dass** ausgehend vom Nebenvolumen (26) mindestens eine Steigleitung (44) ausgebildet und angeordnet ist, die zur Bildung einer Flüssigkeitsverbindung in das Hauptvolumen (25) ragt.

9. Verdampferkartusche (10) nach Anspruch 8, **dadurch gekennzeichnet, dass** ein freies Ende (45) der mindestens einen Strömungskanal (46) aufweisenden Steigleitung (44) zur Aufnahme von Flüssigkeit (18) aus dem Hauptvolumen (25) beabstandet zu der das Hauptvolumen (25) begrenzenden Tankhülle (29) des Vorratstanks (17) ausgebildet ist.

10. Verdampferkartusche (10) nach Anspruch 9, **dadurch gekennzeichnet, dass** innerhalb des Strömungskanals (46) der Steigleitung (44) ein Ventilelement (47) angeordnet ist, das einerseits den Zufluss von Flüssigkeit (18) aus dem Hauptvolumen (25) in das Nebenvolumen (26) ermöglicht und andererseits einen Abfluss von Flüssigkeit (18) aus dem Nebenvolumen (26) in das Hauptvolumen (25) zumindest erschwert.

11. Verdampferkartusche (10) nach einem oder mehreren der Ansprüche 8 bis 10, **dadurch gekennzeichnet, dass** jede Steigleitung (44) im Wesentlichen parallel zum Luftströmungskanal (20) ausgerichtet ist.

12. Verdampferkartusche (10) nach einem oder mehreren der Ansprüche 4 bis 11, **dadurch gekennzeichnet, dass** die rohrförmige Tankhülle (29) eine mundstückseitige Stirnseite (M) und eine bodenseitige Stirnseite (B) aufweist, wobei beide Stirnseiten (B, M) durch ein Deckelelement (33) dichtend verschlossen sind, wobei mindestens ein Deckelelement (33) zum Befüllen des Vorratstanks (17) lösbar an der Tankhülle (29) befestigt ist.

13. Verdampferkartusche (10) nach Anspruch 12, **dadurch gekennzeichnet, dass** das bodenseitig angeordnete Deckelelement (33) lösbar mit der Tankhülle (29) verbunden ist.

14. Verdampferkartusche (10) nach Anspruch 12 oder 13, **dadurch gekennzeichnet, dass** das mundstückseitige Deckelelement (33) einstückig mit der Tankhülle (29) ausgebildet ist, wobei das mundstückseitige Deckelelement (33) als Mundstück (16) geformt ist.

15. Verdampferkartusche (10) nach einem oder mehreren der Ansprüche 4 bis 14, **dadurch gekennzeichnet, dass** der Adapter (31) Mikrobohrungen und/oder Mikroöffnungen (43) aufweist und/oder zusammen mit der Tankhülle (29) und/oder den Deckelelementen (33) bildet, durch die Flüssigkeit (18) aus dem Hauptvolumen (25) in das Nebenvolumen (26) förderbar ist.

16. Verdampferkartusche (10) nach einem oder mehreren der Ansprüche 1 bis 15, **dadurch gekennzeichnet, dass** das Nebenvolumen (26) mindestens teilweise mit einem Flüssigkeit (18) speichernden Material, vorzugsweise mit granulatartigen Körnern, gefüllt ist.

17. Inhalator (11), ausgebildet und eingerichtet zum Inhalieren von mit Wirkstoffen angereichertem Dampf, umfassend einen mindestens eine elektronische Steuereinheit (12) und eine Energiequelle (13) umfassenden Kartuschenträger (14) sowie eine Verdampferkartusche (10), **dadurch gekennzeichnet, dass** die Verdampferkartusche (10) nach einem oder mehreren der Ansprüche 1 bis 16 ausgebildet und eingerichtet ist.

## Claims

1. Vaporizer cartridge (10) suitable for an inhaler (11), comprising at least one storage tank (17) for receiving and storing a liquid (18) and at least one hollow body (19) with a continuous air flow channel (20), wherein the storage tank (17) has at least one access opening (21) to the air flow channel (20), and a vaporizer unit (22) which extends over the entire access opening (21) is arranged in the region of each access opening (21), said vaporizer unit having a wick member (23) directed towards the storage tank (17) and a heating member (24) directed towards the air flow channel (20), wherein the vaporizer unit (22) is configured to be liquid-permeable such that the liquid (18) can be conveyed out of the storage tank (17) through the vaporizer unit (22) in the direction of the air flow channel (20) in a capillary manner at least initially, **characterised in that** the interior of the storage tank (17) is structurally divided into a main volume (25) and at least one auxiliary volume (26), wherein the main volume (25) and the auxiliary volume (26) are fluidically coupled together, and only the auxiliary volume (26) is in direct contact with the vaporizer unit (22), in such a manner that the liquid (18) from the main volume (25) can be brought into contact with the wick member (23) solely and necessarily via the auxiliary volume (26), wherein the interior of the storage tank (17) is divided by a separating wall (27) or similar into the main volume (25) and at least one auxiliary volume (26), wherein the auxiliary volume (26) is in fluid connection with the main volume (25) for receiving liquid (18) therefrom.

2. Vaporizer cartridge (10) according to claim 1, **characterised in that** the auxiliary volume (26) forms a temporary reservoir and establishes a fluid connection between the main volume (25) and the wick member (23) of the vaporizer unit (22).

3. Vaporizer cartridge (10) according to claim 2, **characterised in that** the temporary reservoir shields the wick member (23) from the main volume (25) with the exception of at least one access opening (28) for establishing fluid connection between the auxiliary volume (26) and the main volume (25).

4. Vaporizer cartridge (10) according to one or more of claims 1 to 3, **characterised in that** it comprises a tank shell (29) for forming the main volume (25) of the storage tank (17), an adaptor (31) forming a receiving chamber (30) for forming the auxiliary volume (26) of the storage tank (17) as a temporary reservoir within the main volume (25), as well as a channel element (32) as a hollow body (19) forming the air flow channel (20) or parts thereof.

5. Vaporizer cartridge (10) according to claim 4, **characterised in that** it comprises a carrier element (34) which, on the one hand, has a through-channel (35) for forming the air flow channel (20) or parts thereof and, on the other hand, has a recess (36) for receiving the vaporizer unit (22), wherein the carrier element (34) and the vaporizer unit (22) form a structural unit which is arranged inside the receiving chamber (30) of the adaptor (31) in a sealing manner with respect to the environment.

6. Vaporizer cartridge (10) according to claim 5, **characterised in that** the auxiliary volume (26) forming the temporary reservoir is defined by the inner surface (I) of the receiving chamber (30) of the adaptor (31) directed away from the main volume (25) of the storage tank (17) and the outer surface (A) of the structural unit formed by the vaporizer unit (22) and the carrier element (34), wherein the inner surface (I) of the receiving chamber (30) of the adaptor (31) is configured and adapted to form the auxiliary volume (26) at least partially spaced apart from the outer surface (A) of the structural unit.

7. Vaporizer cartridge (10) according to one or more of claims 4 to 6, **characterised in that** the carrier element (34) and the channel element (32) form a continuous vent (37) with the air flow channel (20) through the whole vaporizer cartridge (10), wherein the carrier element (34) and the channel element (32) are connected to each other in a sealing manner.

8. Vaporizer cartridge (10) according to one or more of claims 4 to 7, **characterised in that**, at least one riser (44), which starts from the auxiliary volume (26) and protrudes into the main volume (25) to form a fluid connection, is configured and arranged.

9. Vaporizer cartridge (10) according to claim 8, **characterised in that** a free end (45) of the riser (44) comprising at least one flow channel (46) for receiving liquid (18) from the main volume (25) is configured at a distance from the tank shell (29) of the storage tank (17) which defines the main volume (25).

10. Vaporizer cartridge (10) according to claim 9, **characterised in that** arranged within the flow channel (46) of the riser (44) is a valve element (47) which, on the one hand, enables the inflow of liquid (18) from the main volume (25) into the auxiliary volume (26) and, on the other hand, at least impedes an outflow of liquid (18) from the auxiliary volume (26) into the main volume (25).

11. Vaporizer cartridge (10) according to one or more of claims 8 to 10, **characterised in that** each riser (44) is oriented substantially parallel to the air flow channel (20).

12. Vaporizer cartridge (10) according to one or more of claims 4 to 11, **characterised in that** the tubular tank shell (29) has an end face (M) on the mouthpiece side and an end face (B) on the base side, wherein both end faces (B, M) are closed by a cover element (33) in a sealing manner, wherein at least one cover element (33) is releasably fastened to the tank shell (29) for filling the storage tank (17).

13. Vaporizer cartridge (10) according to claim 12, **characterised in that** the cover element (33) arranged on the base side is releasably connected to the tank shell (29).

14. Vaporizer cartridge (10) according to claim 12 or 13, **characterised in that** the mouthpiece-side cover element (33) is formed integrally with the tank shell (29), wherein the mouthpiece-side cover element (33) is shaped as a mouthpiece (16).

15. Vaporizer cartridge (10) according to one or more of claims 4 to 14, **characterised in that** the adaptor (31) has and/or, together with the tank shell (29) and/or the cover elements (33), forms micro-holes and/or micro-openings (43) through which liquid (18) can be conveyed from the main volume (25) into the auxiliary volume (26).

16. Vaporizer cartridge (10) according to one or more of claims 1 to 15, **characterised in that** the auxiliary volume (26) is filled at least partially with a liquid (18) storing material, preferably with granular grains.

17. Inhaler (11), configured and adapted for inhaling vapour enriched with active ingredients, comprising at least one electronic control unit (12) and a cartridge carrier (14) comprising an energy source (13) as well as a vaporizer cartridge (10), **characterised in that** the vaporizer cartridge (10) is configured and adapted according to one or more of claims 1 to 16.

## Revendications

1. Cartouche d'évaporateur (10) appropriée pour un inhalateur (11), comprenant au moins un réservoir de stockage (17) pour recevoir et stocker un liquide (18) et au moins un corps creux (19) avec un canal d'écoulement d'air (20) continu, le réservoir de stockage (17) présentant au moins une ouverture d'accès (21) au canal d'écoulement d'air (20) et une unité d'évaporateur (22) s'étendant sur toute l'ouverture d'accès (21) étant agencée dans la zone de chaque ouverture d'accès (21), qui présente un organe de mèche (23) tourné vers le réservoir de stockage (17) et un organe de chauffage (24) tourné vers le canal d'écoulement d'air (20), l'unité d'évaporateur (22) étant configurée sous forme perméable aux liquides, de telle sorte que du liquide (18) puisse être transporté au moins initialement par capillarité hors du réservoir de stockage (17) à travers l'unité d'évaporateur (22) en direction du canal d'écoulement d'air (20), **caractérisée en ce que** le réservoir de stockage (17) est divisé à l'intérieur, de par sa construction, en un volume principal (25) et au moins un volume secondaire (26), le volume principal (25) et le volume secondaire (26) étant couplés l'un à l'autre par le liquide et seul le volume secondaire (26) étant en contact direct avec l'unité d'évaporateur (22), de telle sorte que le liquide (18) provenant du volume principal (25) peut être mis en contact avec l'organe de mèche (23) exclusivement et obligatoirement par l'intermédiaire du volume secondaire (26), le réservoir de stockage (17) étant divisé à l'intérieur par une paroi de séparation (27) ou similaire en le volume principal (25) et au moins un volume secondaire (26), le volume secondaire (26) étant en communication de liquide avec le volume principal (25) pour recevoir le liquide (18) provenant de celui-ci.

2. Cartouche d'évaporateur (10) selon la revendication 1, **caractérisée en ce que** le volume secondaire (26) forme un réservoir intermédiaire et établit une communication de liquide entre le volume principal (25) et l'organe de mèche (23) de l'unité d'évaporateur (22).

3. Cartouche d'évaporateur (10) selon la revendication 2, **caractérisée en ce que** le réservoir intermédiaire isole l'organe de mèche (23) du volume principal (25), à l'exception d'au moins une ouverture d'accès (28) pour établir la communication de liquide entre le volume secondaire (26) et le volume principal (25).

4. Cartouche d'évaporateur (10) selon une ou plusieurs des revendications 1 à 3, **caractérisée en ce qu'**elle comprend une enveloppe de réservoir (29) pour former le volume principal (25) du réservoir de stockage (17), un adaptateur (31) formant un espace de réception (30) pour former le volume secondaire (26) du réservoir de stockage (17) en tant que réservoir intermédiaire à l'intérieur du volume principal (25), ainsi qu'un élément de canal (32) formant le canal d'écoulement d'air (20) ou des parties de celui-ci en tant que corps creux (19).

5. Cartouche d'évaporateur (10) selon la revendication 4, **caractérisée en ce qu'**elle comprend un élément de support (34) qui présente d'une part un canal de passage (35) pour former le canal d'écoulement d'air (20) ou des parties de celui-ci et d'autre part un évidement (36) pour recevoir l'unité d'évaporateur (22), l'élément de support (34) et l'unité d'évaporateur (22) formant une unité structurelle qui est agencée de manière étanche par rapport à l'environnement à l'intérieur de l'espace de réception (30) de l'adaptateur (31).

6. Cartouche d'évaporateur (10) selon la revendication 5, **caractérisée en ce que** le volume secondaire (26) formant le réservoir intermédiaire est délimité par la surface intérieure (I) de l'espace de réception (30) de l'adaptateur (31), détournée du volume principal (25) du réservoir de stockage (17), et par la surface extérieure (A) de l'unité structurelle formée par l'unité d'évaporateur (22) et l'élément de support (34), la surface intérieure (I) de l'espace de réception (30) de l'adaptateur (31) étant configurée et adaptée pour former le volume secondaire (26) au moins partiellement à distance de la surface extérieure (A) de l'unité structurelle.

7. Cartouche d'évaporateur (10) selon une ou plusieurs des revendications 4 à 6, **caractérisée en ce que** l'élément de support (34) et l'élément de canal (32) forment avec le canal d'écoulement d'air (20) une cheminée (37) traversant toute la cartouche d'évaporateur (10), l'élément de support (34) et l'élément de canal (32) étant reliés entre eux de manière étanche.

8. Cartouche d'évaporateur (10) selon une ou plusieurs des revendications 4 à 7, **caractérisée en ce qu'**au moins une conduite montante (44) est configurée et agencée à partir du volume secondaire (26), laquelle fait saillie dans le volume principal (25) pour former une communication de liquide.

9. Cartouche d'évaporateur (10) selon la revendication 8, **caractérisée en ce qu'**une extrémité libre (45) de la conduite montante (44) présentant au moins un canal d'écoulement (46) pour recevoir du liquide (18) provenant du volume principal (25) est configurée à distance de l'enveloppe de réservoir (29) du réservoir de stockage (17) délimitant le volume principal (25).

10. Cartouche d'évaporateur (10) selon la revendication 9, **caractérisée en ce qu'**à l'intérieur du canal d'écoulement (46) de la conduite montante (44), est agencé un élément de soupape (47) qui, d'une part, permet l'arrivée de liquide (18) du volume principal (25) dans le volume secondaire (26) et, d'autre part, rend au moins difficile un écoulement de liquide (18) du volume secondaire (26) dans le volume principal (25).

11. Cartouche d'évaporateur (10) selon une ou plusieurs des revendications 8 à 10, **caractérisée en ce que** chaque conduite montante (44) est orientée essentiellement parallèlement au canal d'écoulement d'air (20).

12. Cartouche d'évaporateur (10) selon une ou plusieurs des revendications 4 à 11, **caractérisée en ce que** l'enveloppe de réservoir (29) tubulaire présente un côté frontal (M) côté embouchure et un côté frontal (B) côté fond, les deux côtés frontaux (B, M) étant fermés de manière étanche par un élément de couvercle (33), au moins un élément de couvercle (33) étant fixé de manière amovible à l'enveloppe de réservoir (29) pour remplir le réservoir de stockage (17).

13. Cartouche d'évaporateur (10) selon la revendication 12, **caractérisée en ce que** l'élément de couvercle (33) agencé côté fond est relié de manière amovible à l'enveloppe de réservoir (29).

14. Cartouche d'évaporateur (10) selon la revendication 12 ou 13, **caractérisée en ce que** l'élément de couvercle (33) côté embouchure est configuré d'une seule pièce avec l'enveloppe de réservoir (29), l'élément de couvercle (33) côté embouchure étant formé en tant qu'embouchure (16).

15. Cartouche d'évaporateur (10) selon une ou plusieurs des revendications 4 à 14, **caractérisée en ce que** l'adaptateur (31) présente et/ou forme conjointement avec l'enveloppe de réservoir (29) et/ou les éléments de couvercle (33) des micro-alésages et/ou des micro-ouvertures (43) par lesquels du liquide (18) peut être transporté du volume principal (25) dans le volume secondaire (26).

16. Cartouche d'évaporateur (10) selon une ou plusieurs des revendications 1 à 15, **caractérisée en ce que** le volume secondaire (26) est au moins partiellement rempli d'un matériau stockant du liquide (18), de préférence de grains de type granulés.

17. Inhalateur (11), configuré et adapté pour inhaler de la vapeur enrichie en substances actives, comprenant un support de cartouche (14) comprenant au moins un organe de commande électronique (12) et une source d'énergie (13), ainsi qu'une cartouche d'évaporateur (10), **caractérisé en ce que** la cartouche d'évaporateur (10) est configurée et adaptée selon une ou plusieurs des revendications 1 à 16.
